# EUROPEAN PATENT SPECIFICATION

(11) **EP 1 702 631 B1**
(45) Date of publication and mention of the grant of the patent: **14.07.2010**
(21) Application number: 06005364.2
(22) Date of filing: 16.03.2006
(51) Int. Cl.: A61L 17/04

(54) **Polyolefin sutures having enhanced durability**
Polyolefin-Nähfäden mit verbesserter Haltbarkeit
Sutures polyoléfines à durabilitées ameliorées

(30) Priority: 16.03.2005 US 81390
(43) Date of publication of application: 20.09.2006
(73) Proprietor: Tyco Healthcare Group LP, Norwalk, CT 06856 (US)
(72) Inventor: Cuevas, Brian J., Middletown, CT 06457 (US); Schiretz, Frank R., Middletown, CT 06457 (US); Cohen, Matthew D., Berlin, CT 06037 (US); Hotter, Joseph, Middletown, CT 06457 (US)
(74) Representative: Soames, Candida Jane

(56) References cited:
- WO-A-97/08238
- WO-A-2005/080495
- US-A1- 2002 177 876

## Description

### TECHNICAL FIELD

The present disclosure relates to surgical sutures, and particularly to a polyolefin suture having improved durability and fray resisting characteristics.

### DESCRIPTION OF RELATED ART

Polyolefin sutures are known in the art. Such sutures are non-absorbable and generally include polypropylene or polymeric combinations of ethylene and propylene. The polymeric components of the polyolefin sutures are generally melt spun to produce filaments for use in fabricating the surgical suture strands. Polypropylene sutures can be advantageously produced as monofilament sutures.

Various methods are known for making polypropylene sutures. For example, U.S. Patent No. 5,217,485 to Liu et al. discloses a process for making a polypropylene monofilament suture by melt extruding the monofilament, stretching the solidified monofilament, then allowing the monofilament to equilibrate, or "rest", prior to annealing.

Polypropylene monofilament sutures are known to exhibit a limited amount of fraying as the suture passes over itself, e.g., when tying knots. Polypropylene sutures have thus become the preferred suture for cardiovascular and vascular surgery.

While the limited amount of fraying exhibited by polypropylene monofilament sutures does not substantially hamper the performance of the suture, there remains room for improvement in the processing and handling characteristics of such sutures.

### SUMMARY

Sutures having improved handling characteristics are made in accordance with the disclosure from a composition containing a polyolefin, a fatty acid diester and a thiodipropionate of the formula

R-OOCCH₂CH₂SCH₂CH₂COO-R,

where R is a C₁₃ or greater alkyl group. The compositions optionally include a synthetic wax and a dye.

Methods for producing sutures from such compositions include the steps of extruding a composition containing a polyolefin, a fatty acid diester and a thiodipropionate and, optionally a synthetic wax and a dye.

### BRIEF DESCRIPTION OF THE FIGURES

FIG. 1 is a diagram of a system utilized for testing the fray resistance of sutures of the present disclosure.
FIG. 2 is a graph depicting cycle to break data of sutures of the present disclosure having varying concentrations of distearylthiodipropionate (DSTDP) and a constant concentration of polyethylene glycol distearate.
FIG: 3. is a graph depicting cycle to break data of sutures of the present disclosure having varying concentrations of polyethylene glycol distearate and a constant concentration of DSTDP.
FIG. 4A and FIG. 4B. are graphs depicting cycle to break data and tensile strength data, respectively, of sutures of the present disclosure having DSTDP, polyethylene glycol distearate, polyethylene wax, and dye, compared with two control sutures.

### DETAILED DESCRIPTION

Sutures in accordance with the present disclosure include a polyolefin in combination with a thiodipropionate and a fatty acid diester. In some embodiments, the sutures of the present disclosure may also contain a synthetic wax.

All composition percentages listed herein shall be understood to be by weight unless otherwise indicated.

Any suitable polyolefin known in the art may be used in producing a suture in accordance with their disclosure. Polyolefins which may be utilized include, for example, polyethylene, polypropylene and copolymers of polyethylene and polypropylene. Blends of polyolefins, (e.g. blends of polyethylene and polypropylene) may also be used. In particularly useful embodiments, polypropylene, which can be isotactic polypropylene or a mixture of isotactic and syndiotactic or atactic polypropylene, is used to form the suture. Crystalline polypropylenes of from 150,000 daltons to 500,000 daltons average molecular weight (Mw) can be especially useful. Suitable isotactic polypropylene resins include those possessing a weight average molecular weight (Mw) of from 200,000 to 350,000 daltons, a number average molecular weight (Mn) of from 50,000 to 180,000 daltons, and a calculated dispersity (Mw/Mn) of from 2.0 to 4.0. The isotactic polypropylene resins can advantageously possess a melt flow index in g/10 min of from 2 to 6 and typically from 3.5 to 4.5. Isotactic polypropylene resins are readily available from numerous commercial sources.

Thiodipropionates suitable for use herein include a propanoic ester backbone, in which the functional side chains are extended with aliphatic groups in place of a hydrogen atom. The general molecular structure of particularly useful thiodipropionates is shown below:

R-OOCCH₂CH₂SCH₂CH₂COO-R

where R is a C₁₃ or greater alkyl group.

In certain embodiments, the thiodipropionate used to form the polyolefin suture as described herein is a thiodipropionate such as 3,3'-thiobis-,dioctadecyl ester propanoic acid, also known as distearylthiodipropionate (DSTDP), or 3,3'-thiobis-,ditridecyl ester propanoic acid, also known as ditridecylthiodipropionate (DTTDP), and combinations thereof. In some embodiments, 3,3'-thiobis-, didodecyl ester propanoic acid, also known as dilaurylthiodipropionate (DLTDP), may be combined with the thiodipropionate having at least 32 carbon atoms. In a particularly useful embodiment, the thiodipropionate used to form the polyolefin suture can be a distearylthiodipropionate such as CARSTAB^{®} DSTDP or MORSTILLE^{®} DSTDP, both sold by Struktol (Stow, Ohio).

While thiodipropionates such as DSTDP have been previously used with polyolefins as antioxidants, in the present disclosure the thiodipropionates are utilized to enhance the fray resisting characteristics of sutures. Without wishing to be bound by any theory, it is believed the thiodipropionates, when mixed with a polyolefin at appropriate concentrations, undergo exudation, i.e., they bloom to the outer surfaces of the polyolefin suture. Once exuded to the outer surfaces of the suture, the thiodipropionates can act as a lubricant and significantly improve the fray resisting characteristics of the polyolefin suture during the suturing process.

The thiodipropionate is present in the suture in amounts ranging from 0.01% to 1.5% by weight of the suture, typically from 0.1% to 1.0% by weight of the suture. In particularly useful embodiments, the thiodipropionate can represent from 0.2% to 0.6% by weight of the suture.

Any fatty acid diester known to one skilled in the art can be used in forming the suture described in the present disclosure. Suitable fatty acids include C₁₀- C₂₆ fatty acids such as stearic, lauric, palmitic, myristic, arachidic, behenic, and similar acids. In some embodiments the fatty acid diester is a diester of a polyalkylene glycol. Suitable polyalkylene glycols include C₂- C₆ alklyene glycols, such as polyethylene glycol and polypropylene glycol.

In particularly useful embodiments, the fatty acid diester can be a fatty acid diester of polyethylene glycol such as, for example, polyethylene glycol distearate (PEG distearate). In particular, a suitable PEG distearate for use in the method described herein has a melting point of from 35° C. to 37° C., an acid value of 5.0, an iodine value of 0.41, and a saponification value of 117.0. Suitable PEG distearates are readily available from numerous commercial sources.

The fatty acid diester can be present in the suture in amounts ranging from 0.01% to 5.0% by weight of the suture. In some embodiments, the fatty acid diester can represent from 0.1 % to 2.0% by weight of the suture. In particularly useful embodiments, the fatty acid diester can represent from 0.2% to 1.5% by weight of the suture.

Sutures produced in accordance with the present disclosure may also have a synthetic wax included therein. Suitable synthetic waxes for use in the present disclosure include polyethylene wax, ethylene copolymer wax, and halogenated hydrocarbon waxes. In some embodiments, the synthetic wax can be a polyethylene wax, such as a low molecular weight polyethylene wax. As used herein, the term "polyethylene wax" refers to both ethylene homopolymers and copolymers of ethylene with α-olefins having a chain length of C₃ -C₁₈, each having a melt viscosity measured at 140° C. of from 5 to 20000 mPa·sec. In certain embodiments, the polyethylene wax is a low molecular weight polyethylene wax having a weight average molecular weight in the range of from 500 to 4000 Daltons, typically from 1000 to 3000 Daltons, more typically 1500 to 2500 Daltons. The polyethylene wax can also be a low density polyethylene, a linear low density polyethylene, a nonlinear low density polyethylene, or a high density polyethylene. As used herein, "LDPE" is a low density polyethylene, usually branched, having a density from 0.91 to 0.94 g/cc; "HDPE" is a high density polyethylene having a density above 0.95 g/cc; and "LLDPE" is a linear low density polyethylene having a density of 0.91 to 0.95 g/cc. Suitable polyethylene waxes are readily available from numerous commercial sources.

Where utilized, the synthetic wax, such as a polyethylene wax, can be present in the suture in amounts ranging from 0.01% to 2.0% by weight of the suture. In some embodiments, the synthetic wax can represent from 0.1 % to 1.5% by weight of the suture. In a particularly useful embodiment, the synthetic wax can represent from 0.2% to 1.0% by weight of the suture.

A synergistic effect may be observed in sutures containing a fray reducing amount of both a thiodipropionate and a fatty acid diester. Such sutures may be found to possess significantly improved fray resisting characteristics compared to either additive alone.

In some embodiments, it may be desirable to include a pigment in the composition of the present disclosure, thereby providing color to the sutures of the present disclosure. The term "pigment" herein is used interchangeably with the term "dye" and refers to such compounds or particles that absorb visible and/or infrared light. Suitable pigments for dyeing polypropylene filaments are known to those skilled in the art. Such pigments include, but are not limited to, carbon black, bone black, copper phthalocyanine dyes, D&C Green No. 6, and D&C Violet No. 2 as described in the handbook of U.S. Colorants for Food, Drugs and Cosmetics by Daniel M. Marrion (1979). Other dyes which may be used include indocyanine green, methylene blue, fluorescein, india ink, Prussian blue, eosins, acridine, iron oxide, and acramine yellow. Those skilled in the art will recognize that detectable moieties may also be utilized with such dyes. Such detectable moieties include, but are not limited to, fluorescers, bioluminescent and chemiluminescent molecules, and the like.

Where utilized, sutures in accordance with the present disclosure may be dyed by adding up to 0.1 % to 1.0 % (by weight of the final suture composition) dye, typically 0.2 % to 0.6 % dye to the resin composition prior to extrusion to form a suture. In one embodiment, a copper phthalocyanine dye such as beta phthalocyaninato (2-) copper dye may be used. Such dyes are readily available from numerous commercial sources and, in some embodiments, may be obtained in a form which includes polyolefins such as polyethylene.

Sutures of the present disclosure may be produced by melt extrusion or "spinning" of the polyolefin combined with the fray reducing amount of the thiodipropionate, optionally in combination with the fray reducing amount of the fatty acid diester, synthetic wax, and/or dye. An exemplary process for manufacturing a suture is described in U.S. Patent Application Pub. No. 2002/0177876.

In one embodiment, the first step in producing sutures of the present disclosure can be performed by directly adding the thiodipropionate to the polyolefin, either prior to or during melting. In some embodiments, the thiodipropionate may be combined with the polyolefin by dry blending. The resulting blend will possess thiodipropionate in an amount as noted above, i.e., an amount ranging from 0.01% to 1.5% by total weight of the composition, typically from 0.1 % to 1.0% by total weight of the composition, more typically from 0.2% to 0.6% by total weight of the composition.

In other embodiments, a composition to be extruded may be prepared by first pre-blending a first portion of polyolefin and thiodipropionate to make what is commonly referred to as a "master batch", which is then combined with a second portion of polyolefin to produce a batch having the desired weight percentages of polyolefin and thiodipropionate.

The amount of thiodipropionate added to the master batch of preblended polypropylene (in pellet or other suitable form) is often referred to as the "letdown" or "letdown ratio". As those skilled in the art will appreciate, the ratio of additional polyolefin to the preblended masterbatch can be adjusted to produce a second batch, and thus a product, having any target percentage of thiodipropionate. In some cases mixing a small quantity of pre-blended polyolefin/thiodipropionate with standard polyolefin pellets may result in better dispersion of the thiodipropionate in the second batch, which may then be heated to form the subsequent polymer melt. The preblended polyolefin can be produced at one facility or operation and formed into a master batch of pellets which can then be stored and/or transferred to the suture fabrication operation. The polyolefin used to make the pre-blended batch of polyolefin /thiodipropionate can have the same characteristics (e.g., molecular weight, melt flow index, etc.) as the standard polyolefin with which the pre-blended batch is combined to form the second batch, which is then heated to form a polymer melt.

When a specific amount or percentage of thiodipropionate is needed to achieve a suture of the present disclosure, a masterbatch can be prepared and then can be "let down" to obtain the desired amount of thiodipropionate in the resulting suture. For example, if the desired thiodipropionate level in the final suture fiber is 1.0% by weight based on the weight of the polyolefin, a 20% masterbatch, for example, can be let down. The term "let down," as used herein, refers to the ratio of:
(w)(z)=(w')(z')
wherein
w is the amount of the masterbatch,
z is the percentage of thiodipropionate in the masterbatch,
w' is the batch size of the polyolefin utilized to produce a suture (in the same units as w), and
z' is the percentage of thiodipropionate in the polyolefin utilized to produce a suture of the present disclosure.

Therefore, in the above example, if w' is 100 pounds (45.4 kg), z' is 1.0% and z is 20%, then 5 pounds (2.3 kg) of the masterbatch is needed to produce a suture having a thiodipropionate concentration of 1.0%. Thus, in this example, the let down ratio would be 5:1, which can also be referred to as a 5% let down.

Where the polyolefin is prepared by the masterbatch process described above, the let down can range from 5% to 100%.

In another embodiment, the first step of the method can be similarly performed by adding both a fray reducing amount of a thiodipropionate and a fray reducing amount of a fatty acid diester to the polyolefin, either prior to or during melting. As described above, in some embodiments it may be advantageous to first form a master batch of preblended polyolefin containing a first portion of polyolefin, thiodipropionate, and fatty acid diester, and then combine the master batch with a second portion of polyolefin to obtain a second batch utilized to form a resin having the desired levels of polyolefin, thiodipropionate and fatty acid diester. In either case, the amount of thiodipropionate will be present in the resulting composition in the amount as noted above, i.e., it can thus range from 0.01 % to 1.5% by weight of the total composition, typically from 0.1 % to 1.0% by weight of the total composition, more typically from 0.2% to 0.6% by weight of the total composition. The amount of fatty acid diester will also range in the amount noted above, i.e., from 0.01% to 5.0% by weight of the total composition, typically from 0.1 % to 2.0% by weight of the total composition, more typically from 0.2% to 1.5% by weight of the total composition.

Dyes or pigments may be added as described above to any of the resin compositions described herein to enhance visualization of the resulting suture in the surgical field.

In still another embodiment, the first step of the method can be similarly performed by adding a fray reducing amount of a thiodipropionate, a fray reducing amount of a fatty acid diester, a synthetic wax, and optionally a dye to the polyolefin, either prior to or during melting. Here also, as described above, in some embodiments a mixture of polyolefin, thiodipropionate, fatty acid diester, synthetic wax, and dye, if any, may be prepared by making a master batch of preblended polyolefin containing a first portion of polyolefin, a thiodipropionate, a fatty acid diester, a synthetic wax, and optionally a dye. The master batch may then be mixed with a second portion of standard polyolefin pellets to provide a second batch which may then be utilized to form a suture having the overall desired level of thiodipropionate, fatty acid diester, synthetic wax, and dye, in amounts noted above. The ratio of standard polyolefin to the preblended polyolefin can be adjusted to produce a product having any target percentage composition of thiodipropionate, fatty acid diester, synthetic wax and/or dye. Mixing a small quantity of pre-blended polyolefin with standard polyolefin pellets may achieve better dispersion of the thiodipropionate, fatty acid diester, synthetic wax and/or dye in the subsequent polymer melt than direct addition of thiodipropionate, fatty acid diester, synthetic wax and/or dye to the polyolefin.

The next step in producing sutures in accordance with the present disclosure involves heating the polyolefin in combination with the thiodipropionate, optionally in combination with the fatty acid diester, polyethylene wax, and/or dye, to form a polymer melt. This melt is then extruded and cooled to form a filament which can then be sent to further processing such as stretching. The melt contains substantially no water or organic solvents, and no substances which would be incompatible with body tissue.

In some embodiments, other additives may be included in the suture material. Suitable additives are known to those skilled in the art and include antioxidants, such as hindered phenols; or vitamin E; antistatic agents; antacids such as metal soaps, including calcium stearate; and additional thioesters, such as DLTDP.

In some embodiments, sutures of the present disclosure can be monofilament sutures. In other embodiments, sutures of the present disclosure can be multifilament sutures. When more than one filament is used to form a suture, the filaments may be braided, twisted, entangled, intertwined or arranged in some other multifilament configuration. Particularly useful braid structures for sutures are the braid structures described in U.S. Pat. Nos. 5,019,093 and 5,059,213.

Sutures as described herein can be used to secure tissue in a desired position.

Wounds may be sutured by approximating tissue and passing the needled suture through tissue to create wound closure. The needle is then typically removed from the suture and the suture tied.

The sutures and methods described herein are illustrated by the following non-limiting examples.

### EXAMPLE 1

Sample monofilament sutures, size 5/0, were prepared in accordance with the general procedures described above. The polymer melt was prepared by combining polypropylene with a polyethylene wax, polyethylene glycol distearate, a dye, and distearylthiodipropionate. The polypropylene possessed certain additives: 0.2% of a mixture of a hindered phenol antioxidant, calcium stearate, and an antistatic agent (Chemstat 273E), and 0.4% DLTDP. Details of the amounts of the above components in the various samples are set forth in Table 1 below.

**TABLE 1**

| Sample | Polypro | | Polethyene Wax | | PEG Distearate | | DSTDP | | Dye | |
|---|---|---|---|---|---|---|---|---|---|---|
| | % | g | % | g | % | g | % | g | % | G |
| 1 | 98.446 | 984.46 | 0.354 | 3.54 | 0.750^{C} | 7.50 | 0 | 0 | 0.450 | 4.50^{B} |
| 2 | 98.146 | 981.46 | 0.354 | 3.54 | 0.750 | 7.50 | 0.300 | 3.00 | 0.450 | 4.50^{B} |
| 3 | 97.596 | 975.96 | 0.354 | 3.54 | 1.000 | 10.00 | 0.600 | 6.00 | 0.450 | 4.50^{B} |
| 4 | 97.946 | 979.46 | 0.354 | 3.54 | 0.750 | 7.50 | 0.500 | 5.00 | 0.450 | 4.50^{B} |
| 5 | 98.046 | 980.46 | 0.354 | 3.54 | 0.750 | 7.50 | 0.400 | 4.00 | 0.450 | 4.50^{B} |
| 6 | 98.046 | 9804.60 | 0.354 | 35.40 | 0.750 | 75.00 | 0.400 | 40.00 | 0.450 | 45.00^{B} |
| 7 | 98.046 | 9804.60 | 0 | 0 | 0.750 | 75.00 | 0.400 | 40.00 | 0.804 | 80.40^{A} |
| 8 | 98.796 | 987.96 | 0 | 0 | 0 | 0 | 0.400 | 4.00 | 0.804 | 8.04^{A} |
| 9 | 98.796 | 987.96 | 0.354 | 3.54 | 0 | 0 | 0.400 | 4.00 | 0.450 | 4.50^{B} |

| | | | | | | | | | | |
|---|---|---|---|---|---|---|---|---|---|---|
| A = EUPOLEN® Blue 70-9001 B = HELIOGEN® Blue K 7090 C = PEG 6000 distearate | | | | | | | | | | |

Sutures were then prepared from the above samples utilizing the parameters in Table 2 below.

| Table 2 | |
|---|---|
| Parameter | Set Point |
| Barrel 1 Temp. (°C.) | 200 ± 5 |
| Barrel 2 Temp. (°C.) | 200 ± 5 |
| Barrel 3 Temp. (°C.) | 200 ± 5 |
| Clamp Temp (°C.) | 200 ± 5 |
| Adaptor Temp. (°C.) | 200 ± 5 |
| Block Temp. (°C.) | 200 ± 5 |
| Pump Temp. (°C.) | 200 ± 5 |
| Die Temp. (°C.) | 200 ± 5 |
| Aux. Die Temp. (°C.) | 210 ±5 |
| Barrel (psi) | 450 - 3000 |
| Pump (psi) | 500 ± 200 |
| Quench (°C.) | 40 ±5 |
| Godet 1 (meters/min, "mpm" | 7.2 (+0.1,-0.2) |
| Godet 2 mpm | 49.5 (± 0.5) |
| Godet 3 mpm | 68.6 (± 0.5) |
| Godet 4 mpm | 50.9 (+1.7, -1.8) |
| Draw 1 (°C.) | 115 ± 3 |
| Draw 2 (°C.) | 130 ± 3 |
| Relax (°C.) | 151 ± 3 |

The physical properties of these sutures were then tested utilizing standard tests known to those skilled in the art, the details of which are set forth below in Table 3.

**TABLE 3**

| PROCEDURES FOR MEASURING PHYSICAL PROPERTIES OF MONOFILAMENT SUTURES | |
|---|---|
| Physical Property | Test Procedure |
| knot-pull strength, kg | U.S.P. XXI tensile strength, sutures (881) |
| straight-pull strength, kg | ASTM D-2256, Instron Corporation |
| elongation, % | ATSM D-2256 |
| tensile strength (modulus), kg/mm² | ASTM D-2256, Instron Corporation Series IX Automated Materials Testing System 1.11 |

The results of these tests are set forth in Table 4 below.

**TABLE 4**

| Sample | Straight Pull Average (kg) | Elongation Average % | Stress at Max- Load Average (Kg/mm²) | Modulus Average (Man Young) (Kg/mm²) | Knot Pull Average Load at Max Load (kg) |
|---|---|---|---|---|---|
| 1 | 1.094 | 43.16 | 64.89 | 216.68 | .7722 |
| 2 | 1.103 | 44.51 | 65.23 | 209.95 | .7613 |
| 3 | 1.093 | 45.86 | 63.71 | 202.60 | .7668 |
| 4 | 1.059 | 46.33 | 65.04 | 207.20 | .7249 |
| 5 | 1.038 | 44.84 | 63.54 | 206.28 | .7187 |

### EXAMPLE 2

Additional samples from Example 1 were subjected to a sterilization step of ethylene oxide (EtO) and their physical properties were tested using the tests set forth in Table 3 above. The results of these tests are set forth in Table 5 below.

**TABLE 5 - STERIZILED SUTURES**

| Sample | Straight Pull Average (kg) | Elongation Average % | Stress at Max-Load Average (Kg/mm²) | Modulus Average (Man Young) (Kg/mm²) | Knot Pull Average Load at Max Load (kg) |
|---|---|---|---|---|---|
| 1 | 1.130 | 38.74 | 67.52 | 274.1 | 0.7266 |
| 2 | 1.090 | 34.05 | 66.00 | 273.9 | 0.8177 |
| 3 | 1.125 | 39.86 | 68.15 | 273.7 | 0.8084 |
| 4 | 1.057 | 36.19 | 65.81 | 267.6 | 0.7958 |
| 5 | 1.068 | 38.55 | 65.57 | 265.75 | 0.7562 |

### EXAMPLE 3

Several studies were performed to evaluate the effects of varying the concentrations of distearylthiodipropionate (0.0%, 0.3% and 0.6%) in 5/0 polypropylene sutures with 0.75% polyethylene glycol distearate.

The sutures were sterilized prior to testing for fray resistance by exposure to EtO, at a temperature of 113° C., for 4 hours.

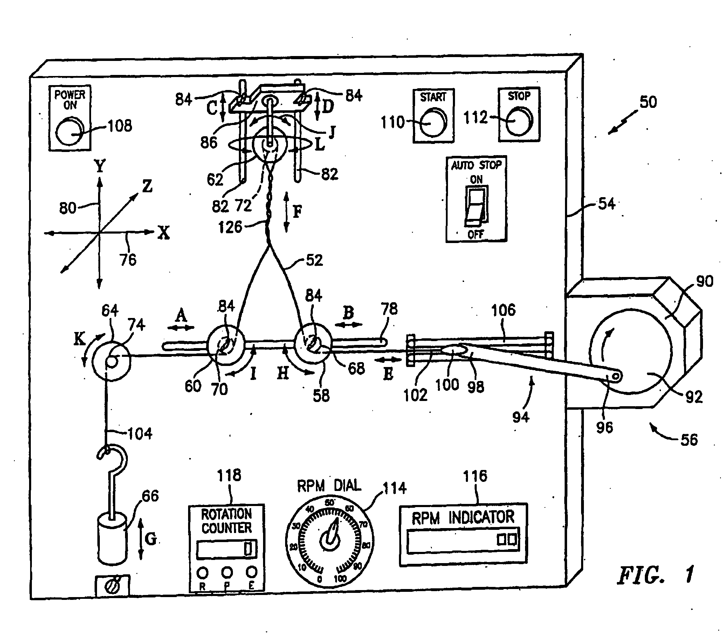

The various sterilized sutures were then exposed to a fray resistance test. The test measured the number of cycles needed to break ("CtB") for each of the various propylene sutures. FIG. 1 illustrates the suture tester, utilized for measuring and evaluating fray resistance properties of the suture.

A suture was cut to an appropriate length. Referring to Fig. 1, a first end of the suture 102 is mounted to the suture gripper 100 of the suture tester 50. The suture 52 is then placed around the first pulley 58 and brought up vertically, making about an 80 degree wrap around the pulley. Then, the suture 52 is guided over the third pulley 62, making about a 300 degree wrap around the pulley. The suture 52 is then guided to the second pulley 60 and then brought over horizontally, making about an 80 degree wrap around the second pulley. Then, the suture 52 is guided over the fourth pulley 64 and brought down vertically, making about a 270 degree wrap around the fourth pulley. A tensioning element 66 is hung at the second end of the suture 104 using an adequate loop portion made thereof, to provide an adequate tension to the suture 52 for the test. Next, the third pulley 62 is rotated to create the desired number of suture wraps and the pulley 62 is then locked into place. The tensioning element was a fifty (50) gram weight for conventional size 5/0 sutures.

As shown in FIG. 1, turning the tester on results in reciprocating action to the suture's first end 102. This reciprocating action caused the suture's wrapped portion 126, and the tensioning element 66 to move substantially up and down as indicated by the doubled headed arrows F and G. In order to facilitate the reciprocating movement of the suture 52, the first, second, third and fourth pulleys 58, 60, 62 and 64 were subjected to subordinate rotational movement to back and forth directions as indicated by arrows H, I, J and K. Thus, the suture 52 rub against itself at its wrapped portion 126 while suitable tension is applied to the suture by the tensioning element 66.

Upon repeated rubbing action, the suture's wrapped portion 126 becomes fragile. Counter 118 displays the number of rubbing cycles or cycles to break at the time the suture fails.

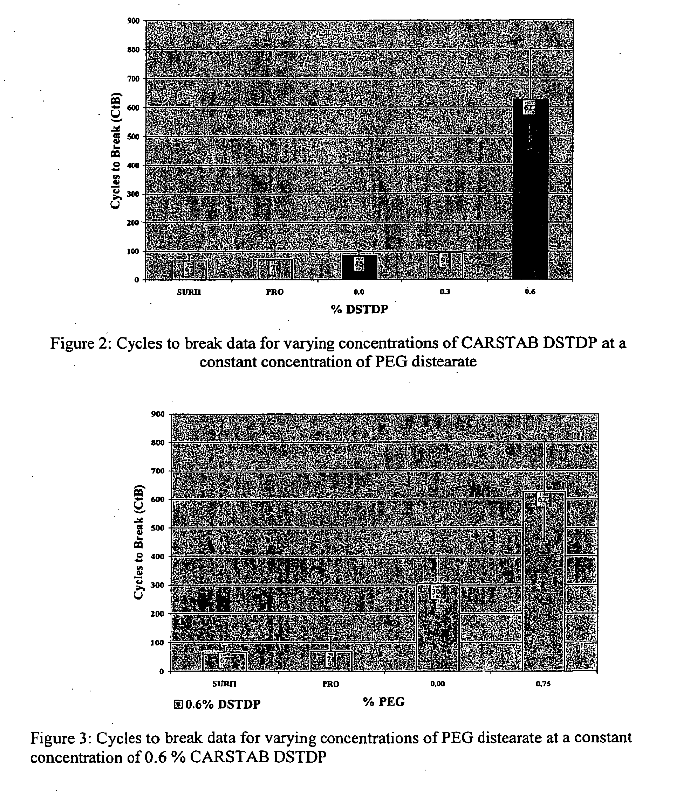

The results of these tests are set forth in Figure 2. The fray testing showed a dramatic increase in cycles to break (CtB) with increased distearylthiodipropionate at a constant concentration of 0.75% PEG-distearate. Specifically, 85 CtB where no DSTDP was added compared to 630 CtB where 0.6% DSTDP was added. (See Figure 2.)

### EXAMPLE 4

Sutures were prepared as in Example 3 above, with the difference being that in this Example the concentration of CARSTAB® DSTDP was held constant (0.6%) while the concentration of PEG distearate varied (0% and 0.75%). Fray testing was performed after sterilization as in Example 3, with the results set forth in Figure 3. As can be seen in Figure 3, an increase in CtB was found with increasing PEG distearate at a constant concentration of 0.6% CARSTAB® DSTDP (300 to 630 CtB).

As can be seen from the above, the addition of thiodipropionate enhanced the fray resistance of polyolefin sutures, with a thiodipropionate-polyolefin suture being more than three times more resistant to fraying than a polyolefin suture (300 CtB vs. 85 CtB)(See Figure 3). Moreover, the addition of a fatty acid diester further enhanced fray resistance, with a thiodipropionate/fatty acid diester/polyolefin suture being more than two times more resistant to fraying than a thiodipropionate/polyolefin suture (630 CtB vs. 300 CtB) and more than seven times resistant to fraying than a polyolefin suture (630 CtB vs. 85 CtB).

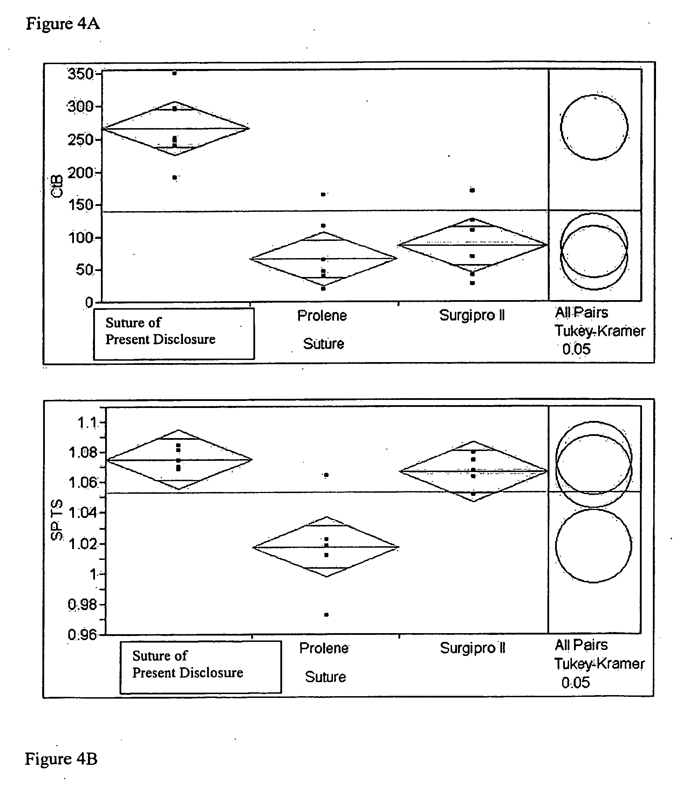

The sutures of the present disclosure were found to have enhanced pigment dispersion on visual examination. The fray resistance, cycles to break (CtB), and the tensile strength of the 5/0 sutures prepared in accordance with one embodiment of the present disclosure are graphically presented in Figures 4A and 4B. Differences in tensile strength and CtB were found when comparing the sutures of the present disclosure with the SURGIPRO II™ and PROLENE™ controls. The sutures of the present disclosure showed enhanced CtB compared with the SURGIPRO II™ and PROLENE™ sutures, without any sacrifice of their already superior tensile strength.

While the above description contains many specifics, these specifics should not be construed as limitations on the scope of the invention, but merely as exemplifications of particularly useful embodiments thereof.

## Claims

1. A suture comprising a sterilized filament comprising a polyolefin, a fatty acid diester and a thiodipropionate of the formula (I) 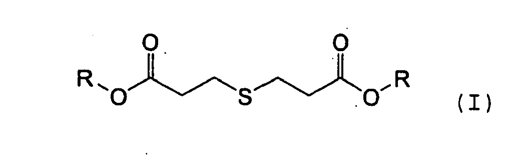 wherein R is an alkyl group having 13 or more carbon atoms, wherein the thiodipropionate is present in an amount of 0.01-1.5 wt.-%, based on the weight of the suture.

2. The suture of claim 1, wherein the polyolefin is selected from polypropylene, polyethylene, copolymers of polyethylene and polypropylene, and combinations thereof.

3. The suture of claim 1 or 2, wherein the thiodipropionate is selected from distearyl thiodipropionate, ditridecyl thiodipropionate and combinations thereof.

4. The suture of any of claims 1-3, wherein the polyolefin comprises polypropylene and the thiodipropionate of formula (I) comprises distearyl thiodipropionate.

5. The suture of any preceding claim, wherein the fatty acid diester is polyethylene glycol distearate.

6. The suture of any preceding claim, wherein the fatty acid diester is present in an amount of 0.01-5.0 wt.-%, based on the weight of the suture.

7. The suture of any preceding claim, wherein the filament further comprises a synthetic wax.

8. The suture of claim 7, wherein the synthetic wax is selected from polyethylene wax, ethylene copolymer wax, and halogenated hydrocarbon waxes.

9. The suture of claim 7 or 8, wherein the synthetic wax is present in an amount of 0.01-2.0 wt.-%, based on the weight of the suture.

10. The suture of any preceding claim, wherein the filament further comprises a dye.

11. The suture of claim 10, wherein the dye is selected from carbon black, bone black, copper phthalocyanine dyes, D&C Green No. 6, D&C Violet No. 2, indocyanine green, methylene blue, fluorescein, india ink, Prussian blue, eosins, acridine, iron oxide, and acramine yellow.

12. The suture of claim 11, wherein the dye is present in an amount of 0.1-1.0 wt.-%, based on the weight of the suture.

13. The suture of any preceding claim, which is a monofilament suture.

14. A method of manufacturing a suture as defined in any preceding claim, which method comprises the steps of
(a) providing a melt containing a polyolefin, a fatty acid diester and a thiodipropionate of formule (I) 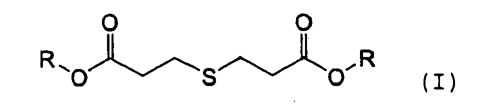 wherein R is an alkyl group having 13 or more carbon atoms, and
wherein the thiodipropionate is present in an amount of 0.01 - 1.5 wt.-%, based on the weight of the suture, and
(b) extruding the melt to form a filament.

15. The method of claim 14, wherein step (a) comprises (a-1) adding a thiodipropionate of formula (I) to a first portion of a polyolefin to form a masterbatch,
(a-2) adding the masterbatch to a second portion of polyolefin to form a second batch; and
(a-3) heating the second batch to provide a melt.

16. The method of claim 15 wherein the polyolefin in the first portion is the same polyolefin as in the second portion.

## Patentansprüche

1. Nahtmaterial, umfassend ein sterilisiertes Filament, umfassend ein Polyolefin, einen Fettsäurediester und ein Thiodipropionat der Formel (I) 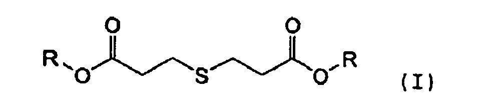 worin R eine Alkylgruppe mit 13 oder mehr Kohlenstoffatomen ist,
wobei das Thiodipropionat in einer Menge von 0,01-1,5 Gew.-%, bezogen auf das Gewicht des Nahtmaterials, vorhanden ist.

2. Nahtmaterial nach Anspruch 1, wobei das Polyolefin aus Polypropylen, Polyethylen, Copolymeren aus Polyethylen und Polypropylen und Kombinationen davon ausgewählt ist.

3. Nahtmaterial nach Anspruch 1 oder 2, wobei das Thiodipropionat aus Distearylthiodipropionat, Ditridecylthiodipropionat und Kombinationen davon ausgewählt ist.

4. Nahtmaterial nach einem der Ansprüche 1 bis 3, wobei das Polyolefin Polypropylen umfasst und das Thiodipropionat der Formel (I) Distearylthiopropionat umfasst.

5. Nahtmaterial nach einem vorangehenden Anspruch, wobei der Fettsäurediester Polyethylenglykoldistearat ist.

6. Nahtmaterial nach einem vorangehenden Anspruch, wobei der Fettsäurediester in einer Menge von 0,01-5,0 Gew.-%, bezogen auf das Gewicht des Nahtmaterials, vorliegt.

7. Nahtmaterial nach einem vorangehenden Anspruch, wobei das Filament außerdem ein synthetisches Wachs umfasst.

8. Nahtmaterial nach Anspruch 7, wobei das synthetische Wachs ausgewählt ist aus Polyethylenwachs, Ethylencopolymerwachs und halogenierten Kohlenwasserstoffwachsen.

9. Nahtmaterial nach Anspruch 7 oder 8, wobei das synthetische Wachs in einer Menge von 0,01-2,0 Gew.-%, bezogen auf das Gewicht des Nahtmaterials, vorliegt.

10. Nahtmaterial nach einem vorangehenden Anspruch, wobei das Filament außerdem einen Farbstoff umfasst.

11. Nahtmaterial nach Anspruch 10, wobei der Farbstoff aus Kohleschwarz, Knochenschwarz, Kupferphthalocyanin-Farbstoffen, D&C Grün Nr. 6, D&C Violett Nr. 2, Indocyaningrün, Methylenblau, Fluorescein, indischer Tusche, Preussischblau, Eosinen, Acridin, Eisenoxid und Acramingelb ausgewählt ist.

12. Nahtmaterial nach Anspruch 11, wobei der Farbstoff in einer Menge von 0,1-1,0 Gew.-%, bezogen auf das Gewicht des Nahtmaterials, vorliegt.

13. Nahtmaterial nach einem vorangehenden Anspruch, das ein Monofilament-Nahtmaterial ist.

14. Verfahren zur Herstellung eines Nahtmaterials, wie es in einem vorangehenden Anspruch definiert ist, wobei das Verfahren die folgenden Schritte umfasst:
(a) Bereitstellen einer Schmelze, die ein Polyolefin, einen Fettsäurediester und ein Thiodipropionat der Formel (I) 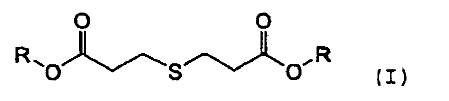 worin R eine Alkylgruppe mit 13 oder mehr Kohlenstoffatomen ist, umfasst und wobei das Thiodipropionat in einer Menge von 0,01-1,5 Gew.-%, bezogen auf das Gewicht des Nahtmaterials, vorliegt;
(b) Extrudieren der Schmelze unter Bildung eines Filaments.

15. Verfahren nach Anspruch 14, wobei Schritt (a) umfasst:
(a-1) Zusetzen eines Thiodipropionats der Formel (I) zu einer ersten Portion eines Polyolefins unter Bildung eines Masterbatchs,
(a-2) Zusetzen des Masterbatchs zu einer zweiten Portion von Polyolefin unter Bildung eines zweiten Batchs und
(a-3) Erwärmen des zweiten Batchs unter Bereitstellung einer Schmelze.

16. Verfahren nach Anspruch 15, wobei das Polyolefin in der ersten Portion das selbe Polyolefin wie in der zweiten Portion ist.

## Revendications

1. Fil de suture, qui comporte un filament stérilisé comprenant une polyoléfine, un diester d'acide gras et un thio-bis(propionate) de formule (I) : 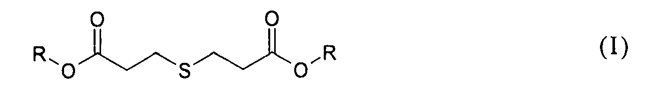 dans laquelle R représente un groupe alkyle comportant au moins 13 atomes de carbone,
dans lequel le thio-bis(propionate) se trouve en une quantité représentant de 0,01 à 1,5 % du poids du fil de suture.

2. Fil de suture conforme à la revendication 1, pour lequel la polyoléfine est choisie parmi les polypropylènes, les polyéthylènes, les copolymères d'éthylène et de propylène, et les combinaisons de tels polymères.

3. Fil de suture conforme à la revendication 1 ou 2, pour lequel le thio-bis(propionate) est choisi parmi le thio-bis(propionate) de distéaryle, le thio-bis(propionate) de ditridécyle, et leurs combinaisons.

4. Fil de suture conforme à l'une des revendications 1 à 3, dans lequel la polyoléfine comprend un polypropylène et le thio-bis(propionate) de formule (I) comprend du thio-bis(propionate) de distéaryle.

5. Fil de suture conforme à l'une des revendications précédentes, dans lequel le diester d'acide gras est un distéarate de polyéthylèneglycol.

6. Fil de suture conforme à l'une des revendications précédentes, dans lequel le diester d'acide gras se trouve en une quantité représentant de 0,01 à 5,0 % du poids du fil de suture.

7. Fil de suture conforme à l'une des revendications précédentes, dans lequel le filament comprend en outre une cire de synthèse.

8. Fil de suture conforme à la revendication 7, pour lequel la cire de synthèse est choisie parmi une cire de polyéthylène, une cire de copolymère d'éthylène et les cires d'hydrocarbures halogénés.

9. Fil de suture conforme à la revendication 7 ou 8, dans lequel la cire de synthèse se trouve en une quantité représentant de 0,01 à 2,0 % du poids du fil de suture.

10. Fil de suture conforme à l'une des revendications précédentes, dans lequel le filament comprend en outre un colorant.

11. Fil de suture conforme à la revendication 10, pour lequel le colorant est choisi parmi les suivants : noir de carbone, noir d'os, colorants de type phtalocyanine au cuivre, Vert D&C n° 6, Violet D&C n° 2, vert d'indocyanine, bleu de méthylène, fluorescéine, encre de Chine, bleu de Prusse, éosines, acridine, oxyde de fer, et jaune d'acramine.

12. Fil de suture conforme à la revendication 11, dans lequel le colorant se trouve en une quantité représentant de 0,1 à 1,0 % du poids du fil de suture.

13. Fil de suture conforme à l'une des revendications précédentes, qui est un fil de suture monofilamentaire.

14. Procédé de fabrication d'un fil de suture conforme à l'une des revendications précédentes, lequel procédé comporte les étapes suivantes :
a) préparer une masse fondue contenant une polyoléfine, un diester d'acide gras et un thio-bis(propionate) de formule (I) : 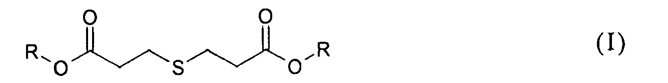 dans laquelle formule R représente un groupe alkyle comportant au moins 13 atomes de carbone,
dans laquelle masse fondue le thio-bis(propionate) se trouve en une quantité qui représentera de 0,01 à 1,5 % du poids du fil de suture ;
b) et extruder cette masse fondue pour en faire un filament.

15. Procédé conforme à la revendication 14, dans lequel l'étape (a) comporte les opérations suivantes :
a-1) ajouter un thio-bis(propionate) de formule (I) à une première portion de polyoléfine, pour en faire un premier mélange ;
a-2) ajouter ce premier mélange à une deuxième portion de polyoléfine, pour en faire un deuxième mélange ;
a-3) et chauffer ce deuxième mélange pour en faire une masse fondue.

16. Procédé conforme à la revendication 15, dans lequel la polyoléfine de la première portion est la même que la polyoléfine de la deuxième portion.
